(19) Europäisches Patentamt
European Patent Office
Office européen des brevets

(11) Veröffentlichungsnummer: **0 335 164 A2**

(12) **EUROPÄISCHE PATENTANMELDUNG**

(21) Anmeldenummer: **89104497.6**

(22) Anmeldetag: **14.03.89**

(51) Int. Cl.[4]: **C07C 101/453 , C07C 101/54 , C07D 231/12 , C07D 209/26 , C07C 69/65 , C07C 69/612 , C07C 69/738 , C07C 69/734 , C07D 333/24 , A61K 31/415 , A61K 31/40**

(30) Priorität: **31.03.88 DE 3811120**

(43) Veröffentlichungstag der Anmeldung:
**04.10.89 Patentblatt 89/40**

(84) Benannte Vertragsstaaten:
**AT BE CH DE ES FR GB GR IT LI LU NL SE**

(71) Anmelder: **Ludwig Merckle GmbH & Co.
chem.-pharm. Fabrik
Dr.-Georg-Spohn-Strasse 7
D-7902 Blaubeuren(DE)**

(72) Erfinder: **Metz, Gunter, Dr.
Auf dem Rucken 29
D-7902 Blaubeuren(DE)**
Erfinder: **Räuchle, Kurt, Dr.
Forstweg 16
D-7901 Blaubeuren-Sonderbuch(DE)**
Erfinder: **Erdmann, Manfred
Josef-Freundorfer Strasse 2A
D-7910 Neu-Ulm 8(DE)**

(74) Vertreter: **Lederer, Franz, Dr. et al
Van der Werth, Lederer & Riederer
Patentanwälte Lucile-Grahn-Strasse 22
D-8000 München 80(DE)**

(54) **Neopentylesterderivate, Verfahren zu ihrer Herstellung und ihre Verwendung als Arzneimittel.**

(57) Neopentylester der bekannten antirheumatisch wirksamen Säuren aus der Gruppe von Diclofenac, Naproxen u.ä. sind neue Verbindungen und besitzen überraschenderweise eine bessere pharmakologische Wirksamkeit als die Säure-Grundkörper. Sie können zur Bekämpfung von Krankheiten des rheumatischen Formenkreises verwendet werden. Sie lassen sich vorteilhaft herstellen durch direkte Veresterung mit einem Alkoholüberschuß ohne Wasserauskreisung.

EP 0 335 164 A2

Xerox Copy Centre

## Neopentylesterderivate, Verfahren zu ihrer Herstellung und ihre Verwendung als Arzneimittel

Die vorliegende Erfindung betrifft Neopentylester der allgemeinen Formel I

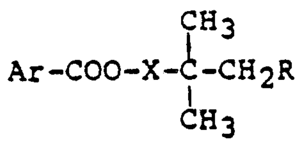

(I)

worin Ar-CO den Rest einer antirheumatisch wirksamen Säure der Gruppe bestehend aus

(a) 2-[(2,6-Dichlorphenyl)amino]-phenylessigsäure (Diclofenac)
(b) 3-(4-Chlorphenyl)-1-phenyl-1H-pyrazol-4-essigsäure (Lonazolac)
(c) 1-(4-Chlorbenzoyl)-5-methoxy-2-methyl-1H-indol-3-essigsäure (Indomethacin)
(d) N-(α,α,α-Trifluor-m-tolyl)anthranilsäure (Flufenaminsäure)
(e) 2-(2-Fluor-4-biphenylyl)-propionsäure (Flurbiprofen)
(f) 2-(4-Isobutylphenyl)-propionsäure (Ibuprofen)
(g) 2-(3-Benzoylphenyl)-propionsäure (Ketoprofen)
(h) 2-(6-Methoxy-2-naphtyl)-propionsäure (Naproxen)
(i) 2-[4-(2-Thenoyl)phenyl]-propionsäure (Suprofen)

X die Gruppe $CH_2$ oder $CH_2COOCH_2$ und
R Wasserstoff oder eine Hydroxygruppe
bedeuten.

Im Falle der Säuren (e) bis (i) sind neben den racemischen Verbindungen auch die optisch aktiven, rechts- und linksdrehenden Isomeren umfaßt.

Die Herstellung der erfindungsgemäßen Neopentylester erfolgt entweder durch direkte Veresterung einer der Säuren (a) bis (i) mit einem Alkohol der allgemeinen Formel II

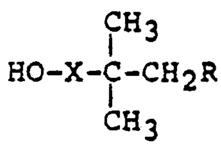

(II)

worin R und X die vorstehend genannte Bedeutung haben, oder durch Umsetzung eines Metallsalzes einer der Säuren (a) bis (i) mit einem Halogenderivat der allgemeinen Formel III

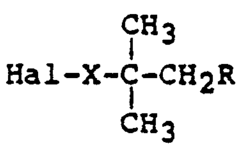

(III)

worin Hal Brom, Jod oder Chlor bedeutet und R und X vorstehende Bedeutung haben.

Verbindungen der allgemeinen Formel I, worin X die Gruppe $CH_2COOCH_2$ bedeutet, sind Doppelester der Struktur

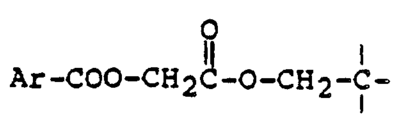

Sofern in der allgemeinen Formel II X für $CH_2$ steht, ist die direkte Veresterung mit den Alkoholen (Neopentylalkohol bzw. 2,2-Dimethyl-1,3-propandiol) die bevorzugte Herstellungsmethode, da die entsprechenden Halogenverbindungen der allgemeinen Formel III mit X = $CH_2$ bei der Synthese zum Teil

schlechtere Ausbeuten ergeben und längere Reaktionszeiten erfordern.

Sofern X die Gruppe $CH_2COOCH_2$ bedeutet, ist die Umsetzung mit den Halogenderivaten der allgemeinen Formel III die bevorzugte Herstellungsmethode, da diese Halogenverbindungen leichter zugänglich sind als die entsprechenden Alkohole und auch schneller reagieren.

Im Falle von X = $CH_2COOCH_2$ ist auch die Veresterung dadurch möglich, daß man die Säuren (a) bis (i) zunächst in die Ester-Säure Ar-$COOCH_2COOH$ umsetzt und mit einem Alkohol der allgemeinen Formel II oder als Metallsalz mit einem Halogenderivat der allgemeinen Formel III zur Reaktion bringt. Entsprechende Ester-Säuren sind bekannt, z.B. von Indomethacin als Acemetacin. Dieses Verfahren ist jedoch dem erfindungsgemäßen Verfahren mit Einführen der gesamten Estergruppe in einer Reaktion unterlegen, da es nicht nur eine zusätzliche und synthetisch aufwendige weitere Stufe beinhaltet, sondern auch in der Gesamtausbeute bezogen auf die Ausgangssäuren (a) bis (i) deutlich schlechter abschneidet.

Die Reaktionen werden bei erhöhter Temperatur gegebenenfalls in einem inerten organischen Lösungsmittel durchgeführt.

Wie überraschend gefunden wurde, kann die direkte Veresterung der Säuren (a) bis (i) unter saurer Katalyse ohne Auskreisen des Reaktionswassers bei mittleren Temperaturen und kurzen Reaktionszeiten durchgeführt werden. Geeignete saure Katalysatoren sind Lewis Säuren, Zinkchlorid, Schwefelsäure, Phosphorsäure, Polyphosphorsäure, Benzolsulfonsäure, p-Toluolsulfonsäure oder Gemische aus Phosphorsäure und p-Toluolsulfonsäure bei molaren Verhältnissen von 0,1 bis 1,0 Mol Katalysator je Mol Säure. Die Reaktion kann hierbei ohne Lösungsmittel durchgeführt werden. Der Alkohol der allgemeinen Formel II wird vorzugsweise mit 8 bis 15 Mol, vorzugsweise 10,5 bis 12 Mol, je Mol Säure eingesetzt. Geeignete Lösungsmittel sind aromatische Kohlenwasserstoffe. Die Reaktionstemperatur beträgt 60° bis 120°, bevorzugt 80° bis 110°, bei Reaktionszeiten von 1 bis 5 Stunden.

Weiterhin kann die gleiche Veresterung in entsprechenden Lösungsmitteln mit den gleichen Katalysatoren nach üblichen Methoden durchgeführt werden, d.h. unter Auskreisen des Reaktionswassers.

Nach an sich bekannten Verfahren werden die Metallsalze der Säuren (a) bis (i) mit den Halogenderivaten der allgemeinen Formel III zu den Estern der Formel I umgesetzt.

Nach der Entfernung des überschüssigen Alkohols oder Halogenderivates durch Ausfällung mit geeigneten Lösungsmitteln oder durch Destillation im Vakuum, wird der erhaltene Rohester umkristallisiert oder im Falle flüssiger Produkte entweder in einer zweistufigen Vakuum-Kurzwegdestillationsanlage destilliert oder mittels präparativer Mitteldruck-Flüssigkeitschromatographie über übliche Sorbentien gereinigt.

Folgende Verfahrensvarianten sollen die Herstellungsmethoden näher beschreiben. Alle Temperaturangaben erfolgen in °C.

Verfahren A - direkte Veresterung ohne Lösungsmittel und ohne Wasserauskreisen

Beispiel 1:

Flufenaminsäure (0,1 Mol) wird unter Zugabe von p-Toluolsulfonsäure Monohydrat (0,1 Mol) bei 80-90° in 2,2-Dimethyl-1,3-propandiol (1,2 Mol) suspendiert und 3 Stunden unter Rühren auf 120° erhitzt. Nach dem Abkühlen auf ca. 80° werden 200 ml Toluol zugesetzt. Der Ansatz wird im Eisbad abgekühlt, wobei der überschüssige Alkohol auskristallisiert. Nach Filtration wird die Toluolphase mit 3 g Natriumcarbonat gelöst in 150 ml Wasser ausgerührt. Die Toluolphase wird nach Abtrennen der Natriumcarbonatlösung mit Wasser (2 x 150 ml) gewaschen, getrocknet und eingedampft. Der Rohester wird mittels Vakuum-Kurzwegdestillation bei 150°C und 1,33 Pa destilliert, wobei in einer Ausbeute von 68 % ein fast farbloses, dickflüssiges Öl erhalten wird. Der erhaltene Flufenaminsäureneopentylglykolmonoester ist dünnschichtchromatographisch rein und zeigt im IR- und NMR-Spektrum die erwarteten Signale. IR(KBr) : 3320, 1680cm$^{-1}$ . NMR($CDCl_3$) : 1.0 ppm (s,6H,$C(CH_3)_2$), 3.37 ppm (S,2H,$CH_2OH$), 4.16 ppm (s,2H,$COOCH_2$).

Verfahren B - direkte Veresterung mit Lösungsmittel ohne Wasserauskreisen

Beispiel 2:

Indomethacin (0,05 Mol) und 2,2-Dimethyl-1,3-propandiol (0,4 Mol) werden in Toluol (150 ml) suspendiert und nach Zugabe von 0,87 g p-Toluolsulfonsäure Monohydrat und 0,67 g o-Phosphorsäure (85 %)

unter Rühren 1,5 Stunden auf 80° erhitzt. Der Ansatz wird im Eisbad abgekühlt und der ausgefallene überschüssige Alkohol abfiltriert. Die Toluolphase wird zunächst mit 6%-iger Natriumcarbonatlösung (200 ml) ausgerührt, mit Wasser gewaschen und nach dem Trocknen über Natriumsulfat eingedampft. Der Rückstand wird aus wäßrigem Isopropanol umkristallisiert, wobei 69 % reiner Ester vom Fp 93-94° erhalten werden.

Verfahren C - direkte Veresterung mit Lösungsmittel und Auskreisen des Reaktionswassers

Beispiel 3:

Lonazolac (0,1 Mol) und 1,1-Dimethylpropanol (0,2 Mol) werden in Toluol (100 ml) suspendiert und nach Zugabe von 2 g p-Toluolsulfonsäure Monohydrat 1 Stunde am Wasserabscheider unter Rückfluß erhitzt. Die Toluolphase wird mit 10%-iger Natronlauge (100 ml) extrahiert, mit Wasser (2 x 50 ml) gewaschen, getrocknet und eingedampft. Der Rückstand wird mittels Vakuum-Kurzwegdestillation bei 200° und 1,33 Pa destilliert. Der Lonazolacneopentylester wird in einer Ausbeute von 77 % erhalten und kristallisiert beim Stehenlassen (Fp 48°).

Verfahren D - Umsetzung mit Halogenderivat $ClCH_2COOCH_2(CH_3)_3$

Beispiel 4:

Flurbiprofen Natrium (0,05 Mol) wird in Dimethylformamid (100 ml) suspendiert und tropfenweise mit Chloressigsäureneopentylester (0,05 Mol) versetzt. Der Ansatz wird 3 Stunden auf 80° erhitzt und danach das Lösungsmittel in Vakuum abdestilliert. Der Rückstand wird in 3%-iger Natronlauge (200 ml) suspendiert und der im Eisbad auskristallisierte Rohester abfiltriert. Nach. Kristallisation aus Isopropanol (60 ml) werden 53 % reiner Ester vom Fp 58-59° erhalten.

Nach diesen Verfahren werden folgende weitere in den Tabellen 1 und 2 erfaßten Ester hergestellt.

Tabelle 1

| Ester durch direkten Umsatz mit $HO-X-C(CH_3)_2-CH_2R$ | | | | | | |
|---|---|---|---|---|---|---|
| Ar-CO | $-OCH_2C(CH_3)_2CH_2OH$ | | | $-OCH_2C(CH_3)_3$ | | |
| | Beisp. | Ausb.% | Fp °C/Kp °C | Beisp. | Ausb.% | Fp °C/Kp °C |
| Säure | | | Pa | | | Pa |
| Ketoprofen | 5 | 73 | 180/1,33 | 12 | 67 | 170/1,33 |
| Naproxen | 6 | 66 | 65 | 13 | 63 | 58-59 |
| Lonazolac | 7 | 68 | 210/1,33 | 3 | 60 | 48 |
| Flurbiprofen | 8 | 70 | 57 | 14 | 80 | 77 |
| Suprofen | 9 | 56 | 200/1,33 | 15 | 48 | 42 |
| Diclofenac | 10 | 55 | - [1]) | 16 | 73 | 170/1,33 |
| Ibuprofen | 11 | 74 | 125/10,6 | 17 | 63 | 120/1,33 |
| Indomethacin | 2 | 69 | 93-94 | 18 | 52 | 89 |
| Flufenaminsäure | 1 | 68 | 150/1,33 | 19 | 65 | 100/1,33 |

[1]) Reinigung durch Mitteldruck-Säulenchromatographie

Tabelle 2

| Ester durch Reaktion mit Hal-X-$C(CH_3)_2$-$CH_2R$ | | | |
|---|---|---|---|
| Ar-CO | -$OCH_2COOCH_2C(CH_3)_3$ | | |
| | Beisp. | Ausb.% | Fp °C/Kp °C |
| Säure | | | Pa |
| Ketoprofen | 20 | 55 | 68-70 |
| Naproxen | 21 | 57 | 53-54 |
| Lonazolac | 22 | 61 | 75 |
| Flurbiprofen | 4 | 63 | 58 |
| Suprofen | 23 | 56 | 94 |
| Diclofenac | 24 | 55 | 54 |
| Ibuprofen | 25 | 53 | - 1) |
| Indomethacin | 26 | 58 | 79 |
| Flufenaminsäure | 27 | 62 | - 1) |

1) Reinigung durch Mitteldruck-Säulenchromatographie

Es ist bekannt, daß nicht steroidale entzündungshemmende Säuren, darunter auch Säuren aus der Gruppe (a) bis (i), durch Veresterung in sogenannte Prodrugs umgewandelt werden können, die günstigere Eigenschaften als die Säure selbst besitzen. Dies bezieht sich auf die Verträglichkeit, insbesondere wird das ulcerogene Potential der Säuren im Magen-Darm-Trakt wesentlich reduziert (J. Pharm. Pharmacol. 32, 795, 1980). Diesem günstigen Befund, der an Methyl- und Ethylestern erhoben wurde, steht als ungünstiger Aspekt gegenüber, daß diese Ester in den entzündungshemmenden Eigenschaften denen der freien Säure weit unterlegen sind. So sind die Ester von Naproxen (J. Med. Chem. 13, 203, 1970) oder von Fenbufen (J. Pharm. Sci. 66, 466, 1977) deutlich geringer wirksam, was einer ungenügenden Freisetzung der aktiven Säure nach Resorption im Organismus zugeschrieben wird.

Tatsächlich wurde in vitro gezeigt, (J. Med. Chem., 30, 451, 1987), daß der Ethylester von Naproxen oder die Methylester von Ketoprofen und Indomethacin in 80%-igem Humanplasma zwischen 20 und 150 Stunden zu 50 % hydrolysieren.

Wie nun gefunden wurde, besitzen die erfindungsgemäßen Ester, wie erwartet, ein reduziertes ulcerogenes Potential, überraschenderweise aber eine entzündungshemmende Wirkung, welche der ihrer Säuren überlegen ist. In Bezug auf o.a. Kenntnisse war die erhöhte Aktivität dieser Ester nicht zu erwarten.

Die entzündungshemmende Wirkung wurde mit ausgewählten Verbindungen jedes Estertyps im Vergleich zum jeweiligen Standard im konventionellen Rattenpfotenoedemtest nach oraler Applikation untersucht. Die Ergebnisse sind in Tab. 3 zusammengefaßt. Weiterhin wurde mit einigen Verbindungen die entzündungshemmende Aktivität nach dermaler Applikation am Kaolin-induzierten Pfotenoedem der Ratte untersucht. Für den Test wurden die Substanzen in einer Mischung aus Ethanol/Aceton/Dimethylacetamid gelöst und im konstanten Volumen 30 Min. vor und 150 Min. nach Injektion von Kaolin (0,1 ml 10%-ige Suspension in Wasser) appliziert. Das Pfotenvolumen wurde 2, 4 und 6 Stunden nach Kaolininjektion gemessen. Die Ergebnisse sind in Tabelle 4 zusammengefaßt.

Wie die Ergebnisse nach oraler Applikation (Tab. 3) zeigen, sind die Ester dem jeweiligen Standard in der Wirkung überlegen und in Bezug auf die im Ester-Prodrug enthaltene Menge der aktiven Säure bei deutlich geringerer Dosierung wirksam. Diese überlegene Wirkung ist auch bei topischer Anwendung (Tab. 4) vorhanden.

Die erfindungsgemäßen Ester werden in Arzneimitteln zur oralen, rektalen und dermalen Anwendung eingesetzt. Geeignete oral verabreichbare Formulierungen sind Tabletten, Dragees, Weich- und Hartgelatinekapseln, die unter Verwen dung üblicher Hilfsstoffe, nach an sich bekannten Methoden hergestellt werden. Zur rektalen Anwendung geeignet sind Suppositorien oder Rektalkapseln, welche die erfindungsgemäßen Ester gelöst im Trägermaterial enthalten. Zur dermalen Anwendung geeignet sind Salben und Cremes vom Typ Öl in Wasser (O/W) oder Wasser in Öl (W/O) unter Verwendung von üblichen Salbengrundlagen und Hilfsstofen, wie Fettkomponenten, Emulgatoren und Stabilisatoren, ferner Gele unter Verwendung von Tylose, Carboxymethylcellulose, Salze von Acrylsäurepolymerisaten oder Acrylsäuren-Acrylamid Copolymerisaten als Strukturbildner in wäßrigen oder alkoholischwäßrigen Zubereitungsformen, sowie Lösungen in dermal verträglichen Lösungsmitteln, wie Alkoholen, Alkohol-Wasser-Mischungen oder Fettalkoholen und -estern. Ebenfalls geeignet sind dermale Formulierungen, die zusätzlich Penetrationsvermittler enthalten, wie Dimethylsulfoxid in Konzentrationen von 10 bis 40 %, oder die Hornschicht erweichende Stoffe, wie Harnstoff mit 5 bis 10 % Anteil in der Formulierung.

Die erfindungsgemäßen Arzneimittel enthalten die neuen Neopentylester im allgemeinen in einer Konzentration von 1 bis 10 % bei dermalen, sowie 25 bis 500 mg bei oralen und rektalen Formulierungen.

Die erfindungsgemäßen Neopentylester werden insbesondere zur Bekämpfung von Krankheiten des rheumatischen Formenkreises verwendet. Die Dosierung erfolgt in der gleichen Größenordnung wie bei den entsprechenden Säuren, beziehungsweise etwas niedriger wegen der erhöhten Wirksamkeit. Im allgemeinen ist eine Einzeldosierung von 25 bis 500 mg, je nach Aktivität der zugrundeliegenden Standardsäure, angebracht, die bis zu dreimal täglich verabfolgt wird.

Tabelle 3 Entzündungshemmende Wirkung im Pfotenoedemtest Ratte nach oraler Applikation.

| Estertyp Ar-CO | Substanz/ Beisp.Nr. | mg/kg | Hemmeffekt[1)] % | S/T[2)] |
|---|---|---|---|---|
| 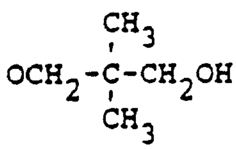 | Suprofen | 50 | 20 | 50 |
| | 9 | 25 | 43 | 18.8 |
| | | 10 | 34 | 7.5 |
| | Ibuprofen | 50 | 35 | 50 |
| | 11 | 50 | 47 | 30.5 |
| | Flufenaminsäure | 50 | 33 | 50 |
| | 1 | 50 | 55 | 38 |
| | | 25 | 38 | 19 |
| | Ketoprofen | 38 | 44 | 38 |
| | 5 | 10 | 33 | 7.5 |
| 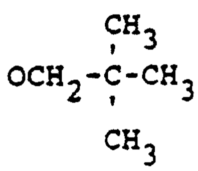 | Suprofen | 50 | 20 | 50 |
| | 15 | 5 | 37 | 3.9 |
| | Diclofenac | 100 | 38 | 100 |
| | 16 | 50 | 36 | 40 |
| | Ibuprofen | 50 | 35 | 50 |
| | 17 | 25 | 43 | 18.7 |
| | Flurbiprofen | 50 | 40 | 50 |
| | 14 | 1 | 35 | 0.78 |
| 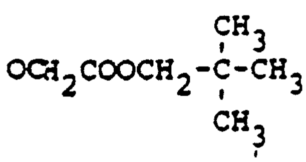 | Diclofenac | 100 | 38 | 100 |
| | 24 | 50 | 33 | 34.9 |
| | Flurbiprofen | 50 | 40 | 50 |
| | 4 | 25 | 33 | 17.5 |
| | Lonazolac | 25 | 38 | 25 |
| | 22 | 15 | 46 | 10.6 |

Fortsetzung Tabelle 3

1) der Hemmeffekt wurde 3 Stunden nach Applikation des Provokators Carrageenan gemessen; Substanzapplikation 1 Stunde vor Carrageenan.

2) S/T = Verhältnis in mg/kg von Standardsäure zum Gehalt Säure in jeweiliger Esterdosierung.

Tabelle 4

| Substanz/Ester Bsp.Nr. | mg/kg | % Hemmung | | |
|---|---|---|---|---|
| | | 2h | 4h | 6h |
| Lonazolac | 10 | 0 | 3.1 | 30.0*** |
| 3 | 12.3[1)] | 18.2* | 31.6*** | 34.3*** |
| Flufenaminsäure | 10 | 0 | 19.4** | 23.2** |
| 1 | 13.2[1)] | 2.2 | 23.7*** | 36.8*** |
| Diclofenac | 10 | 0 | 36.2*** | 37.6*** |
| 16 | 12.4[1)] | 19.2* | 40.1*** | 43.2*** |

[1)] Dosierung entspricht 10 mg/kg der Standardsäure
Signifikanzen bezüglich Kontrollgruppe: * p<0,05 ** p<0,01 *** p<0,001

**Ansprüche**

1. Neopentylester der allgemeinen Formel 1

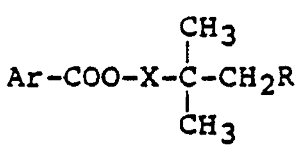

(I)

worin Ar-CO den Rest einer antirheumatisch wirksamen Säure ausgewählt aus der Gruppe bestehend aus Diclofenac, Lonazolac, Indomethacin, Flufenaminsäure, Flurbiprofen, sowie den racemischen und optisch aktiven Formen von Ibuprofen, Ketoprofen, Naproxen und Suprofen,
X die Gruppe $CH_2$ oder $CH_2COOCH_2$ und
R Wasserstoff oder eine Hydroxygruppe
bedeuten.

2. Neopentylester von Diclofenac der Formel

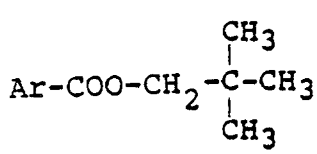

3. Neopentylester von Naproxen der Formel

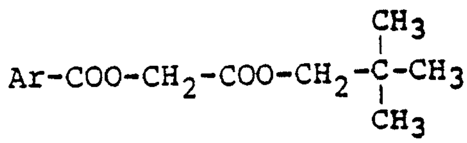

4. Neopentylester von Flufenaminsäure der Formel

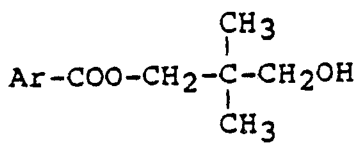

5. Neopentylester von Lonazolac der Formel

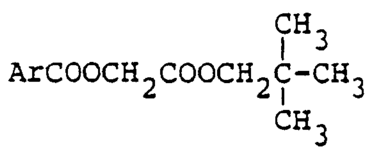

6. Neopentylester von Indomethacin der Formel

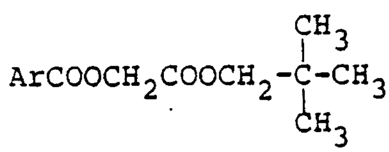

7. Neopentylester von Suprofen der Formel

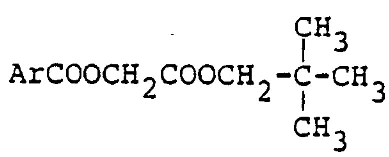

8. Verfahren zur Herstellung der Neopentylester nach Anspruch 1, dadurch gekennzeichnet, daß man

a) eine der in Anspruch 1 genannten Säuren mit einem Alkohol der allgemeinen Formel II

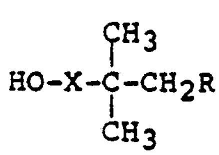

(II)

worin X und R die in Anspruch 1 genannte Bedeutung besitzen, bei erhöhter Temperatur, gegebenenfalls in einem inerten organischen Lösungsmittel und in Gegenwart eines sauren Katalysators unter Wasserabscheidung verestert oder

b) ein Metallsalz der in Anspruch 1 genannten Säuren mit einem Halogenderivat der allgemeinen Formel III

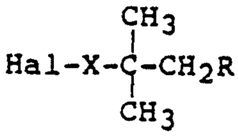

(III)

worin Hal Chlor, Jod oder Brom bedeutet und R und X die in Anspruch 1 genannte Bedeutung haben, bei erhöhter Temperatur in einem geeigneten organischen Lösungsmittel umsetzt.

9. Verfahren nach Anspruch 8 a), dadurch gekennzeichnet, daß man eine der in Anspruch 1 genannten Säuren mit einem Alkohol der allgemeinen Formel II bei einer Temperatur von 80 bis 120 °C ohne azeotropes Entfernen des Reaktionswassers verestert, den überschüssigen Alkohol durch Zugabe von inerten organischen Lösungsmitteln ausfällt oder durch Vakuumdestillation entfernt und den erhaltenen Rohester umkristallisiert oder im Falle flüssiger Produkte in einer Kurzweg-Vakuumdestillationsanlage destilliert und/oder mittels Mitteldruck-Flüssigkeitschromatographie reinigt.

10. Verfahren nach Anspruch 9, dadurch gekennzeichnet, daß man die Säure und den Alkohol im Mol-Verhältnis von 1:(8-15), vorzugsweise 1:(10,5-12) einsetzt.

11. Arzneimittel enthaltend einen Neopentylester nach einem der Ansprüche 1-7 und übliche galenische Hilfsstoffe.

12. Arzneimittel nach Anspruch 11 zur dermalen Anwendung enthaltend zusätzlich Dimethylsulfoxid in einer Konzentration von 10-40 % und gegebenenfalls 5-10 % Harnstoff.

13. Verwendung der Neopentylester nach einem der Ansprüche 1-7 zur Herstellung von oralen, rektalen und dermalen Arzneimitteln für die Bekämpfung von Krankheiten des rheumatischen Formenkreises.